# EUROPEAN PATENT SPECIFICATION

(11) **EP 3 054 872 B1**
(45) Date of publication and mention of the grant of the patent: **17.07.2019**
(21) Application number: 14851512.5
(22) Date of filing: 10.10.2014
(51) Int. Cl.: A61B 17/68

(54) **METHODS AND APPARATUS FOR BONE RESHAPING**
VERFAHREN UND VORRICHTUNG ZUR KNOCHENUMFORMUNG
PROCÉDÉS ET APPAREIL POUR LE REFAÇONNAGE OSSEUX

(30) Priority: 11.10.2013 US 201361890087 P
(43) Date of publication of application: 17.08.2016
(73) Proprietor: NuVasive Specialized Orthopedics, Inc., San Diego, CA 92121 (US)
(72) Inventor: BILGER, Luke, A., Irvine, CA 92618 (US); ROSCHAK, Edmund, J., Irvine, CA 92618 (US); SANDERS, Roy, W., Tampa, FL 33629 (US)
(74) Representative: Boult Wade Tennant LLP
(86) International application number: PCT/US2014/060131
(87) International publication number: WO 2015/054630

(56) References cited:
- WO-A2-2012/112396
- US-A- 5 466 261
- US-A- 5 626 579
- US-A1- 2003 078 582
- US-A1- 2004 030 395
- US-A1- 2005 234 448
- US-A1- 2008 294 269
- US-A1- 2008 294 269
- US-A1- 2009 088 766
- US-A1- 2011 196 435
- US-A1- 2011 196 435
- US-A1- 2013 041 288
- US-B1- 6 673 079

## Description

### BACKGROUND

The present invention relates to orthopaedic methods and devices for the gradual correction of bone deformities. In particular, the present invention relates to any of a variety of bone reshaping procedures, including the lengthening of a bone, the shortening of a bone, the healing of a fracture, the changing of a bone angle, the rotation of a bone, the adjustment of the curvature or torsion of a bone, the realignment or repositioning of a joint or a vertebra, the reforming or supporting the shape of the spinal column, or combinations thereof.

External fixation devices, adjustable in length and angular attitude, are commonly utilized for correcting certain angular and longitudinal defects of long bones of limbs. Such fixation devices essentially comprise clamps which hold groups of bone screws inserted in the portions of the bone affected by defects, such clamps being slidably mounted on elements or guides longitudinally positionable externally to the limb to be treated.

The correction is normally carried out gradually with the aid of compression/distraction devices which act on the mobile clamps while the bone callous regenerates itself permitting its manipulation until the desired correction is obtained.

For example, in limb lengthening, the bone is surgically divided into two segments and wires and half pins are inserted into bone segments above and below the surgical bone cut and attached to rings of a rigid framework interconnected by struts or telescopic connection rods. The rigid framework is used to gradually push the two bone segments apart longitudinally over a period of time (e.g., one millimeter a day). This allows the bone to gradually form in the gap between bone segments created by this distraction technique. Once the desired amount of lengthening is achieved (e.g., 5-6 cm), the external apparatus is stabilized into a fixed position and left on the bone segments until complete mineralization of the newly formed bone (e.g., 3-6 months, depending on the nature of pathology and amount of lengthening).

Similarly, in deformity correction, the bone is surgically divided (usually at the apex of the deformity) into two segments and wires and half pins are inserted into bone segments above and below the surgical bone cut and attached to rings of a rigid framework. Opposite rings of the rigid framework are connected together by threaded rods with attached uni-planar or multi-planar hinges and angular distractor that is used to gradually push the two bone segments apart angularly over a period of time.

One common fixation device is a circular metal structure known as the Ilizarov apparatus. The Ilizarov apparatus, when used for limb lengthening or deformity correction, consists of several rings or arches that are placed externally around the limb and attached to surgically separated bone segments using wires and half pins. For limb lengthening, the opposite rings are interconnected directly by three or four threaded or telescopic rods that are regularly adjusted in length and allowed for gradual separation of bone segments longitudinally. For angular deformity correction, the opposite rings of the Ilizarov apparatus are connected by a pair of hinges that provide an axis of rotation for bone segments and an angular distractor that gradually pushes two rings and associated bone segments apart.

Another common external fixation device is the Taylor Spatial Frame, which is a hexapod type external fixation device based on a Stewart platform but shares many components and features of the Ilizarov apparatus. The Taylor Spatial Frame generally consists of two external fixation rings attached to bone segments by wires and half pins and connected together by 6 telescopic struts with multi-planar hinges located at both ends of the strut. Each strut may be lengthened or shortened as necessary to either pull two interconnected ring segments towards each other or push them apart. Adjustment of strut length allows manipulating with bone segments acutely or gradually in 6 axes (e.g., lengthening/shortening external/internal rotation, anterior/posterior horizontal translation, medial/lateral horizontal translation, anterior/posterior angular translation, and medial/lateral angular translation) to perform limb lengthening and correct angular, translational and rotational deformities simultaneously.

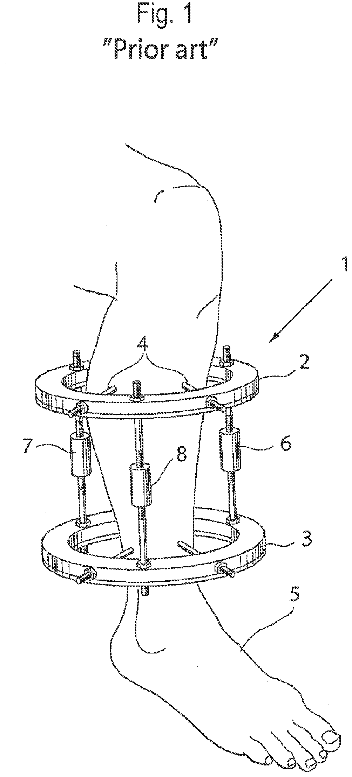

One such prior art external frame system is schematically illustrated in FIG. 1. An external fixator 1 of the type frequently referred to as an Ilizarov apparatus, here consists of two rings 2, 3 having pins 4 attached to and stabilizing the tibia or fibula in the lower leg 5 of a patient. The distance between the rings 2, 3 of the fixator 1 can be adjusted by manually turning threaded cylinders 6, 7, 8 on struts connecting the rings.

Although widely utilized, the foregoing bone reshaping techniques are accompanied by several disadvantages. One is that bone reshaping is normally accomplished by first performing an osteotomy to sever the bone, followed by external fixation to maintain the severed bone components in a desired orientation. The patient must thereafter recover both from the osteotomy as well as from the invasion of soft tissue to attach the frame to the bone. Another disadvantage of the foregoing systems is the need for the external frame to be securely connected to the bone, via percutaneous pins or rods. As the frame must be worn for an extended period of time, the percutaneous support structures become a significant source of potential infection.

Thus, notwithstanding the efforts of the prior art, there remains a need for an improved technology for remodeling bone.

US 2011/0196435 describes a method and device for bone adjustment by imparting a bone reshaping force on the bone. The apparatus includes first and second attachment points configured to be attached to first and second locations on the bone and to apply the bone reshaping force and an adjustable portion configured to be non-invasively actuated to generate the bone reshaping force.

### SUMMARY

The present invention provides an apparatus configured for reshaping bone without creating an osteotomy as set out in claim 1. In the following description a method of reshaping a bone is provided. The method of reshaping a bone comprises the steps of: providing a bone reshaping device, having a first attachment point and a second attachment point; attaching the first attachment point to a first site on an unmodified bone having a deformity; and attaching the second attachment point to a second site on a bone, wherein at least a portion of the deformity is in between the first and second attachment points. The bone reshaping device is actuatable to impart a bone reshaping force on the bone.

A method of reducing a curvature in a bone is also provided. The method of reducing a curvature in a bone comprises: identifying a bone having a longitudinal curvature, the curvature having a convex side and a concave side; and attaching a bone reshaping device to the bone. The bone reshaping device is configured to apply a force to the bone, without performing an osteotomy, to gradually straighten the bone.

Also, a method of changing the shape of a long bone without creating an osteotomy is provided. The method of changing the shape of a long bone without creating an osteotomy comprises; providing at least one bone reshaping device having a first end with a first attachment point and a second end with a second attachment point, wherein the bone reshaping device is configured to create at least one force between the first end and the second end; and implanting the at least one bone reshaping device completely under the skin of a subject. Implanting the at least one bone reshaping device comprises; attaching the first attachment point at a first location on the long bone; and attaching the second attachment point at a second location on the long bone.

### BRIEF DESCRIPTION OF THE DRAWINGS

FIG. 1 shows a prior art external fixation system, having percutaneous pins anchored to the bone.
FIG. 2 is a schematic view of a bone reshaping system, having an implant attached to the bone without an osteotomy, and an external wireless control.
FIG. 3 shows a system where two implanted devices are attached to a bone without an osteotomy.
FIG. 4 shows a detailed view of a system for bone adjustment.
FIGS. 5a and 5b illustrate the straightening of a bone, or how the curvature of a bone can be adjusted, using devices in combination with an osteotomy.
FIG. 5c shows how the curvature of a bone, here a femur, can be adjusted using intramedullary devices.
FIGS. 5d and 5e illustrate how an intramedullary device can be used to adjust the torsion of a bone, here illustrated as the femur, following osteotomy.
FIGS. 6a through 6b illustrate bone reshaping implants attached to reshape deformities in the spine.
FIG. 7 is a side view of a bone reshaping device in accordance with the present invention, adapted for axial lengthening and shortening.
FIG. 8a illustrates a longitudinal cross-sectional view of the device of FIG. 7, taken along the lines 8a-8a.
FIG. 8b illustrates a detailed view of the lengthening device of FIG. 8a, from the area of circle 8b.
FIG. 8c illustrates a cross-sectional view of the device of FIG. 7, taken along the line 8c-8c.
FIG. 9a illustrates a perspective view of some of the internal components of the device of FIG. 7.
FIG. 9b illustrates a lip seal configured for use in the device of FIG. 7.
FIG. 10 illustrates a detail view of several internal components of the drive mechanism of the device of FIG. 7.
FIG. 11 is a schematic illustration of the components of an external control device, for wirelessly controlling the implantable device of FIG. 7.

### DETAILED DESCRIPTION

The present invention is defined by the claims and concerns implantable devices for the adjustment of a bone, comprising at least one elongated device adapted to be implanted in attachment to the bone. The device includes a first attachment point for attachment to a first site on the bone and a second attachment point for attachment to a second site on the bone. The first and second attachment points are spaced apart by the distance of a desired treatment zone.

The device further comprises an adjustment component for adjusting at least one rotational, axial or other spatial relationship between the first and second attachment points, which will apply a reshaping force to the treatment zone of the bone.

The adjustment device is constructed to be implanted completely subcutaneously, so that no part of the device needs to extend through the skin. The adjustment component enables postoperative adjustment, to change the applied reshaping force. The implantable device for the adjustment of a bone is adapted to receive wireless control signals, non-invasively transmitted from an external control for adjusting the adjustment component to control reshaping to the bone.

Unlike conventional bone reshaping technologies, the method described herein may be accomplished on a bone which is unmodified throughout the treatment zone. Thus, rather than functioning to control two segments of bone separated by an osteotomy, the method reshapes bone by loading the bone and allowing the bone to reform in response to that load under normal processes. Depending upon the desired clinical result, it may be desirable to accomplish some modification of bone within the treatment zone. This modification may include providing one or two or four or more perforations through at least a portion of the cortical bone. Modifications may also include providing one or two or more grooves or scores, oriented in a manner to facilitate the desired bone reshaping.

In a device according to some embodiments, the shaping force applied to the bone from the device can be configured to cause the lengthening of a bone, the shortening of a bone, the healing of a fracture, the changing of a bone angle, the rotation of a bone, the adjustment of the curvature or torsion of a bone, the reshaping of a bone, the realignment or repositioning of a joint or a vertebra, the reforming or supporting the shape of the spinal column, or a combination thereof.

In some embodiments, the device is configured to accomplish at least one of: bringing at least two bone-parts defining a fracture closer to each other for a period of time having a beneficial influence on the initiation of the healing process, and bringing the at least two bone-parts defining a fracture away from each other for a period of time having a beneficial influence on the formation of bone, during the healing process.

Examples of conditions contemplated as potentially treatable in accordance with some embodiments include congenital deformities (birth defects), such as congenital short femur; fibular hemimelia (absence of the fibula, which is one of the two bones between the knee and the ankle); hemiatrophy (atrophy of half of the body); and Ollier's disease (also known as multiple endochondromatosis, dyschondroplasia, and endochondromatosis); developmental deformities, such as neurofibromatosis (a rare condition which causes overgrowth in one leg); and bow legs, resulting from rickets (rachitis) or secondary arthritis; post-traumatic injuries, such as growth plates fractures; malunion or non-union (when bones do not completely join, or join in a faulty position after a fracture); shortening and deformity; and bone defects; infections and diseases, such as osteomyelitis (a bone infection, usually caused by bacteria); septic arthritis (infections or bacterial arthritis); and poliomyelitis (a viral disease which may result in the atrophy of muscles, causing permanent deformity); reconstruction after removal of tumors; short stature, such as achondroplasia (a form of dwarfism where arms and legs are very short, but torso is more normal in size); constitutional short stature; and others as may be apparent to those of skill in the art in view of the disclosure herein.

The methods described herein enable the subcutaneous implantation of the bone reshaping device, followed by complete healing of the surgical access site, without the need for any transdermal pins or other structures required by the conventional external fixation devices. The reshaping device is controlled by an external controller which enables application and adjustment of a bone reshaping force over time. In particular applications, the methods enable bone reshaping without any fracturing or disruption of the underlying bone. Instead, controllable force applied to the bone over a therapeutic period of time causes bone remodeling in response to the applied load, without the need for osteotomy.

Once the device is attached to the bone, such as directly attached, the deformity may be characterized by studying the postoperative x-rays. The angular, translational, rotational, and length deformity values may then be entered into specialized software. The software may then produces a "prescription" of device adjustments that the patient can implement via the external controller. The device(s) may be adjusted at least daily by the patient until the correct alignment is achieved. Correction of the bone deformity may take on the order of 3-4 weeks. Once the deformity has been corrected, the device may be surgically removed or left in place.

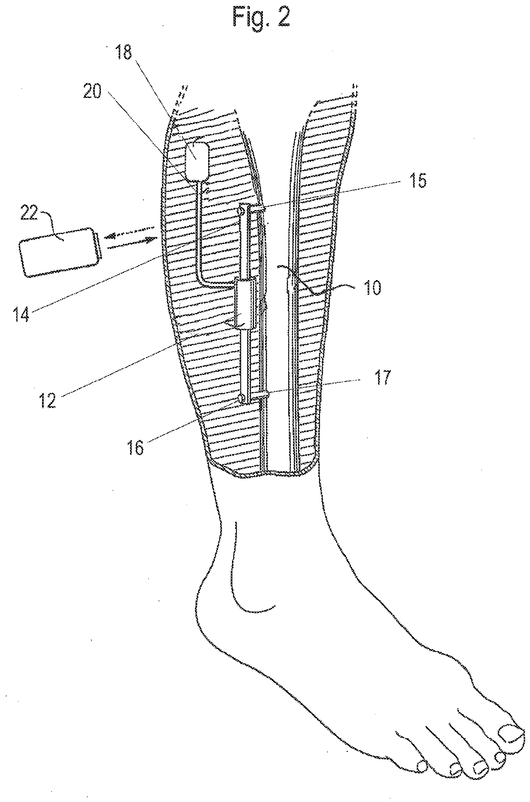

Referring to FIG. 2, there is schematically illustrated an unmodified tibia 10 having a bone reshaping implant 12 attached thereto. Bone reshaping implant 12 may be configured for axial expansion for compression, and/or torsion or other movement necessary to achieve the desired clinical result.

Bone reshaping implant 12 is provided with at least a first attachment point 14 and a second attachment point 16 for attachment to the bone 10. In the illustrated device, first and second attachment points 14 and 16 may be apertures, for receiving a first bone screw 15 and a second bone screw 17. Attachment of the implant 12 to the bone 10 may be accomplished in any of a variety of ways, such as by utilizing one or two or more bone screws at each of the first and second attachment points. Alternatively, the first and second attachment points may be connected to a plate, adhesively secured to the bone, secured to a collar or ring which surrounds at least a portion or potentially encircles the bone, or other attachment structure having sufficient structural integrity to achieve the desired clinical result.

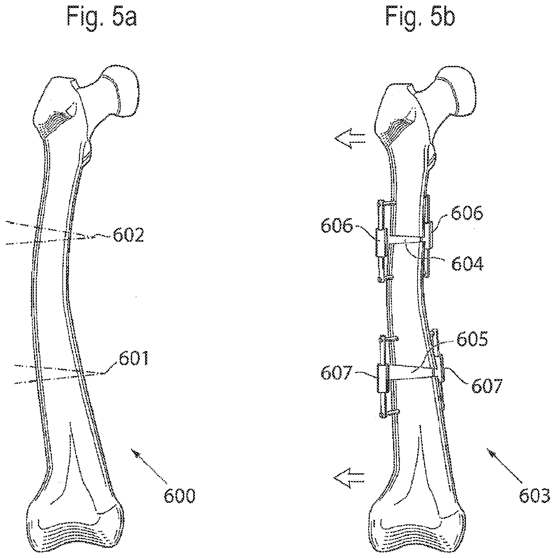

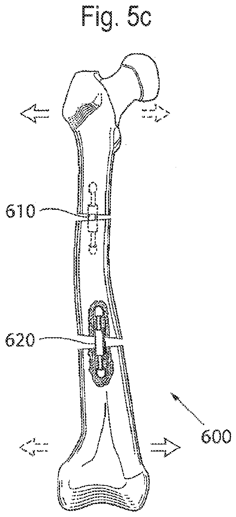

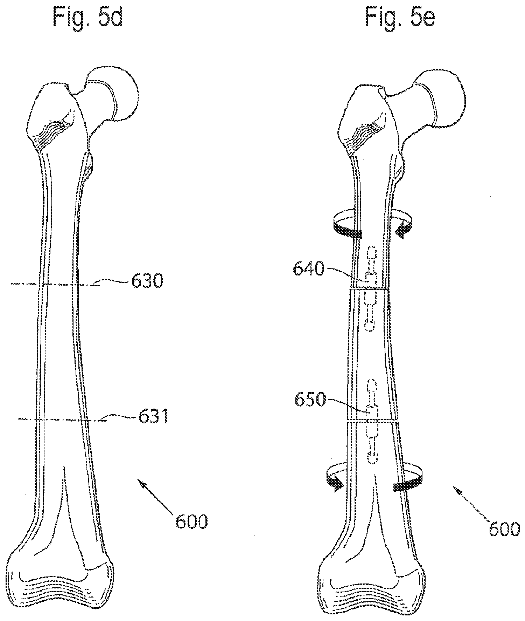

The distance between first attachment point 14 and second attachment point 16, or the longitudinal dimension of the treatment zone, may vary considerably based on the bone to which the bone reshaping implant 12 is being applied. For example, longer bones may compensate a longer treatment zone - that is, a more massive a bone, such as the femur, can accommodate a longer treatment zone. When the bone reshaping implant 12 is being applied to long bones, such as the femur, tibia, radius, or ulna (or any other long bone), the distance between first attachment point 14 and second attachment point 16 can be in the range of about 2.5-30.5 cm (1-12 inches), about 5.1-25.4 cm (2-10 inches), about 7.6-20.3 cm (3-8 inches), and about 10.2-15.2 cm (4-6 inches) in some embodiments. It should be noted that the treatment zone may be tailored to the morphology of any bone defect that is to be corrected. Furthermore, as discussed below, with respect to FIG. 5, multiple treatment zones may exist. Such treatment zones may individually be in the range of about 2.5-30.5 cm (1-12 inches), about 5.1-30-25.4 cm (2-10 inches), about 7.6-20.3 cm (3-8 inches), and about 10.2-15.2 cm (4-6 inches) in some embodiments.

The portion of bone located between the first bone screw 15 and second bone screw 17 is the treatment zone, over which forces generated by the bone reshaping implant 12 will be distributed. Although not illustrated, the bone 10 in between the first bone screw 15 and second bone screw 17 will normally contain one or more bone deformities, which are sought to be corrected.

Shortening the bone reshaping implant 12, and the distance between the first bone screw 15 and the second bone screw 17 (a negative length of travel and decrease in length), applies compressive forces to the treatment zone thereby generally decreasing the length of the treatment zone. In some indications in which compressive forces are desired, to be discussed in more detail below, the device may have a length of travel in the range of about (-1.3)-(-12.7) cm ((-0.5)-(-5.0) inches), about (-2.5)-(-10.2) cm ((-1)-(-4) inches), about (-3.8)-(-7.6) cm ((-1.5)-(-3) inches), and about (-5.1)-(-6.4) cm ((-2)-(-2.5) inches). Lengthening the bone reshaping implant 12, and the distance between the first bone screw 15 and the second bone screw 17 (a positive length of travel and increase in length), applies distractive forces to the treatment zone thereby generally increasing the length of the treatment zone. In some indications in which distractive forces are desired, to be discussed in more detail below, the device may have a length of travel in the range of about 1.3-12.7 cm (0.5-5.0 inches), about 2.5-10.2 cm (1-4 inches), about 3.8-7.6 cm (1.5-3 inches), and about 5.1-6.4 cm (2-2.5 inches).

Optionally, the electrical and mechanical components that drive the bone reshaping implant 12 may be contained within a single housing. Alternatively, one or more modules 18 may be provided, in communication with the bone reshaping implant 12 by a cable 20. Cable 20 may include electrical, optical, fluid, or other conduit, depending upon the functionality of the implant. Optional module 18 may include any of a variety of components which are desired to be maintained off board the bone reshaping implant 12, such as power supply, radiofrequency transmission or receptional electronics, pumps, motors, or others depending upon the desired device configuration.

Although the system illustrated in FIG. 2 shows a single bone reshaping implant 12, two or three or more implants may be secured to a given bone, depending upon the complexity of the deformity or other clinical requirements.

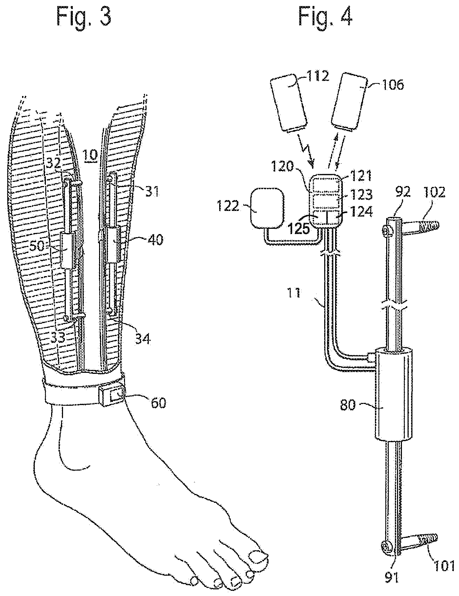

This is illustrated schematically in FIG. 3 where a tibia 10 is shown, supported by two devices 40, 50, both devices being attached to anchoring devices 31, 32, 33, 34 attached to the bone.

In some cases involving the implantation of more than one bone reshaping device, the mechanical characteristics of the multiple bone reshaping devices will be determined based upon the desired clinical outcome. For example, in an implementation such as that illustrated in FIG. 3, the first and second bone reshaping devices 40 and 50 may both provide axial compression, or they may both provide axial tension. Alternatively, a first of the two bone reshaping devices 40 and 50 may provide tension and the other of the two bone reshaping devices 40 and 50 may provide compression. Any of the attachment points to the bone may be rotated circumferentially relative to the bone to add a rotational or torsional component to the bone reshaping force.

In some cases, the one or more bone reshaping devices may be left implanted in the subject after the completion of treatment. In other embodiments, the one or more bone reshaping devices may be removed from the subject after completion of treatment. The length of treatment generally depends on the treatment parameters and desired outcome. For example, distraction of a heavy long bone (such as a femur) can require more time than distraction of a slim long bone (such as an ulna). Additionally, distractions of long length can take more time than distractions of short length. In some embodiments, depending on the treatment parameters and desired outcome, the length of time the one or more bone reshaping devices may be left in a subject can be in the range of about 2-24 weeks, about 3-20 weeks, about 4-16 weeks, about 5-12 weeks, and about 6-10 weeks. In other embodiments, the length of time the one or more bone reshaping devices may be left in the patient can be greater than 24 weeks.

FIG. 4 shows a schematic view of an extension device, here schematically shown as a hydraulic device 80 having two actuators 91, 92 attached to two anchoring devices 101, 102 which can be conventional pins or screws, suitable for inserting in bone. The hydraulic device is in fluid communication through a tube 11 to a hydraulic power unit 120 supplying pressurized hydraulic fluid, which in turn communicates with a control unit 124. Optionally, the control unit 124 also supplies the hydraulic power unit with energy. The hydraulic power unit may comprise a reservoir and a pump or a hydrophore type of pre-pressurized expansion reservoir or any other hydraulic solution. The control unit, energy source, reservoir, pump or motor may all be implanted separate or together in any combination.

The power unit 120 can further be connected to or comprise a hydraulic pump 121 associated with a reservoir 122 containing of a fluid used to regulate the pressure of the device 80. The pump is thus adapted to pump the hydraulic fluid in or out from the device 80 in order to adjust the pressure in the device and the position of the actuators 91, 92.

The power unit 120 can also comprise a rechargeable battery 123 chargeable from the outside by an external power supply/charger unit 112 sending wireless energy.

The adjustment can be controlled by an electronic remote control unit 124 adapted to receive and transmit signals from a transmitter/receiver 106 located outside the body of a treated patient.

The hydraulic device preferably comprises a device positioning system such as a fluid volume or flow measurement or any other sensor input to see the position of the adjustment device. A sensor sensing elongation, for example a capacitance sensor or impedance sensor or any sensor sensing movement or a specific position is preferably provided, here indicated as 125, a sensor communicating with the control unit 124.

Alternatively the schematic FIG. 4 may also instead show a mechanical device 80. In such a case a mechanical wire is outlined as 11 adapted to operate the mechanical device. The power unit 120 may in such a case instead comprise a motor 121 a servo 123 and as before the control unit 124 and sensor 125. The rechargeable power supply may instead be indicated by the unit 122. The motor may of course be placed directly in the mechanical unit 80, wherein the mechanical wire 11 instead is an electrical wire.

The force exerted by the adjustment device may be a longitudinal force, adjusting the angle or curvature of the bone. This is illustrated in FIG. 5a schematically showing a frontal view of the right femur 600 exhibiting a curvature deviating from the natural form of this bone. The curvature may be due to a congenital disease or other condition. The dashed lines 601, 602 indicate how the bone can be fractured, preferably by sawing (e.g. osteotomy). In one example, wedge shaped parts are removed and the bone divided into sections, here illustrated as three sections.

FIG. 5b shows how these three sections of the femur 603 are repositioned to a desired orientation, i.e. a straighter bone. The fracture zones 604, 605 are then used as growth zones in order to compensate for the loss of length due to the removal of bone. Devices 606, 607 according to the invention are then attached via actuators and anchoring devices to the sections, ensuring their position and exerting force to achieve an elongation by distractive osteogenesis. The arrows illustrate schematically that the parts of the bone can be adjusted in relation to each other, for example by adjusting the angle or orientation of the parts.

Two or more anchoring devices may be adapted to engage the cortical part of the bone at each of the first and second attachment points. The two or more anchoring devices may be adapted to engage the bone from the inside of the intramedullar cavity.

The at least two anchoring devices may be chosen from a pin, a screw, an adhesive, a barb construction, a saw-tooth construction, an expandable element, combinations thereof or other mechanical connecting members.

The force exerted by the adjustment device may be a longitudinal force, extending the length of the bone.

The force exerted by the adjustment device may be a longitudinal force, adjusting the angle or curvature of the bone.

The compression and/or distraction force exerted by the adjustment device on the treatment zone may be at least about 44 N (10 lbs). The force required is determined by the indication and may vary. For example, when treating a massive long bone (such as the femur) without first creating an osteotomy, a significant amount of force could be required. By contrast, when treating a slim long bone (such as the ulna) in which an osteotomy or fracture has been created, it is likely that only a relatively low amount of force is required. In some indications, the compressive and/or distraction force is in the range of about 111-890 N (25-200 lbs), about 222-778 N (50-175 lbs), about 334-667 N (75-150 lbs), and about 445-556 N (100-125 lbs). It is generally desirable in some cases to use a force less than 890 N (200 lbs), regardless of the bone being treated, as greater forces may generate highly localized forces that can have adverse effects in some cases.

The force exerted by the device may apply torque to the bone, adjusting the torsion of the bone along its longitudinal axis.

The torque exerted by the adjustment device on the treatment zone may be at least about 0.56 Nm (5 in•lb). As just discussed with respect to compressive and distractive forces, the torque required is determined by the indication and may vary widely. In some indications, the torque required in about 5.65 Nm (50 in•lbs). In some other indications, the torque is in the range of about 1.13-14.69 Nm (10-130 in•lbs), about 2.26-12.43 Nm (20-110 in•lbs), about 3.39-10.17 Nm (30-90 in•lbs), about 4.52-7.91 Nm (40-70 in•lbs), and about 5.08-6.78 Nm (45-60 in•lbs).

A related example is illustrated in FIG. 5c where a deformed bone 600 is cut at one or two locations, 601 and 602, each cut preferably being wedge shaped in order to allow for the straightening of the bone, and devices 610 and 620 are inserted into the medullar cavity. Similarly as in FIG. 5b, the arrows illustrate schematically that the parts of the bone can be adjusted in relation to each other, for example by adjusting the angle or orientation of the parts.

The force exerted by the device may apply torque to the bone, adjusting the torsion of the bone along its longitudinal axis. The magnitude of rotation required to correct a defect in a bone can vary substantially by indication, but will generally be less than about 30 degrees. In some indications the magnitude of rotation to be applied is in the range of about 1-30 degrees, about 3-25 degrees, about 5-20 degrees, about 7-15 degrees, and about 10 degrees. An intermedullary embodiment is illustrated in FIGS. 5d and 5e, where a bone 600 is cut along the dashed line 630 and optionally along one or more lines, exemplified as 631. One or more implantable device or devices 640 and 650 according to the invention are inserted into the medullar cavity. The arrows indicate that one or several parts of the bone can be adjusted, for example rotated in relation to a joint, or to a section of the bone.

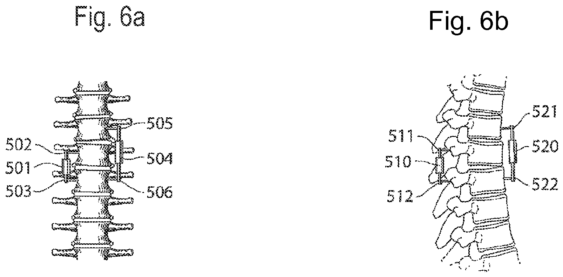

A device as described herein can also be applied to the adjustment of the spine, such as adjustment of a curvature of the spine. FIG. 6a-6b illustrates an example where a device is applied to the adjustment of the curvature of the spine. FIG. 6a is a posterior view of the vertebrae of the lower back, schematically showing two devices 501, 504 attached to opposite sides of the lumbar spine. For illustration purposes, one device 501 is shown attached to two adjoining vertebrae by two anchoring devices 502, 503 whereas another device 504 is shown attached to two non-adjoining vertebrae by two anchoring devices 505, 506. It should be understood that a single device may be used to treat the spine. That is, one device 501 may be attached to the spine and used to alter a conformational characteristic of the spine. The device 501 may be attached to adjacent vertebral bodies. Alternatively, the device 501 may be attached to a first and a second vertebral body having one intervening vertebral body, two intervening vertebral bodies, three intervening vertebral bodies, or more intervening vertebral bodies. Finally, in some cases, the device 501 may be attached to a single vertebral body to alter a conformational characteristic of that single vertebral body. In the same way, more than one device 501 may be used to treat the spine.

FIG 6b is a lateral detail schematically showing two devices 510, 520 attached to opposite sides of the spine by anchoring devices 511, 512, 521, 522. For illustration purposes, one device acts on adjoining vertebrae, whereas the other device acts on non-adjoining vertebrae. This embodiment can be used to adjust the curvature of the spine, such as to relieve a herniated lumbar disc or the like. As described above, depending on the treatment parameters and desired outcome, either one or both of the devices 510 or 520 may be attached to adjacent vertebral bodies: it should be noted that device 510 may be attached to entirely separate adjacent vertebral bodies from those to which device 520 is attached. Alternatively, the device 510 may be attached to first and second vertebral bodies having at least one intervening vertebral body and the device 520 may be attached to a third and fourth vertebral bodies having at least one intervening vertebral body. Between the first and second and third and fourth vertebral bodies (independently of each other), there may be one intervening vertebral body, two intervening vertebral bodies, three intervening vertebral bodies, or more intervening vertebral bodies.

While FIGS 6a-6b illustrate two devices attached to portions of the thoracic spine, it is understood that the devices and methods disclosed herein may be applied to other regions of the spine, including the vertebrae of the cervical, thoracic, lumbar, and lumbar sacral spine (spinal region). Additionally, a device may have attachment points (e.g., 502 & 503) spanning any number of intervening vertebrae (a vertebral span), including, but not limited to one vertebra, two vertebrae, three vertebrae, four vertebrae, and five vertebrae or more vertebrae. Depending on the desired treatment parameters, any number of devices may be used, for example, one device, two devices, three devices, four devices, five devices, six devices, or even more devices. To achieve the desired result, any combination of spinal region, number of devices, and vertebral span may be used.

The wireless energy-transmission device 112 may transmit a carrier signal for carrying the wireless energy signal. Such a carrier signal may include digital, analogue or a combination of digital and analogue signals. In this case, the wireless energy signal includes an analogue or a digital signal, or a combination of an analogue and digital signal.

Optionally, the control signal may comprise any or combination of magnetic energy, kinetic energy, sound energy, chemical energy, radiant energy, electromagnetic energy, (e.g. RF) photo energy, or thermal energy.

The external energy-transmission device 112 may also include a wireless remote control having an external signal transmitter for transmitting a wireless control signal for non-invasively controlling the apparatus. In some embodiments, the control signal is received by an implanted signal receiver incorporated into an implanted energy-transforming device, such as a rechargeable battery 123. In other embodiments, the control signal is received by the sensor 125. In yet other embodiments, the control signal is received by a dedicated and separate receiver.

The wireless control signal may include a frequency, amplitude, or phase modulated signal or a combination thereof. Alternatively, the wireless control signal includes an analogue or a digital signal, or a combination of an analogue and digital signal. Alternatively, the wireless control signal comprises an electric or magnetic field, or a combined electric and magnetic field.

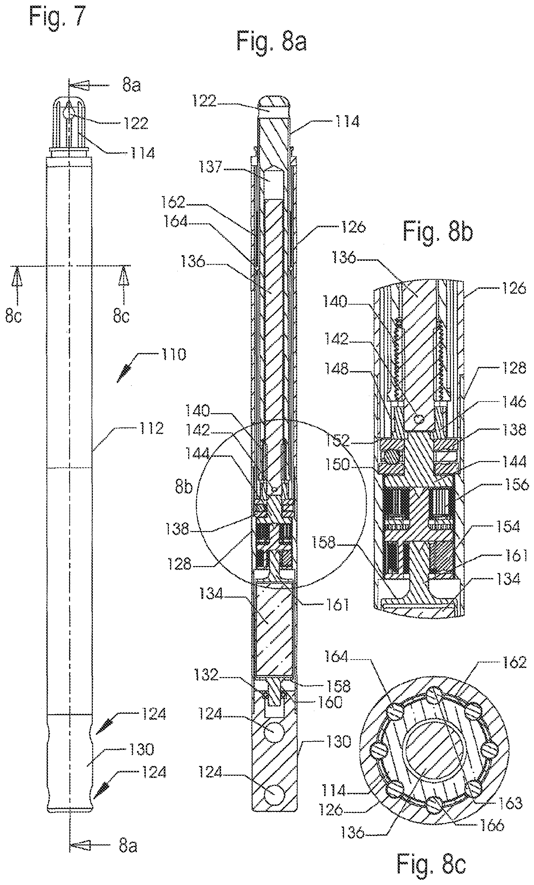

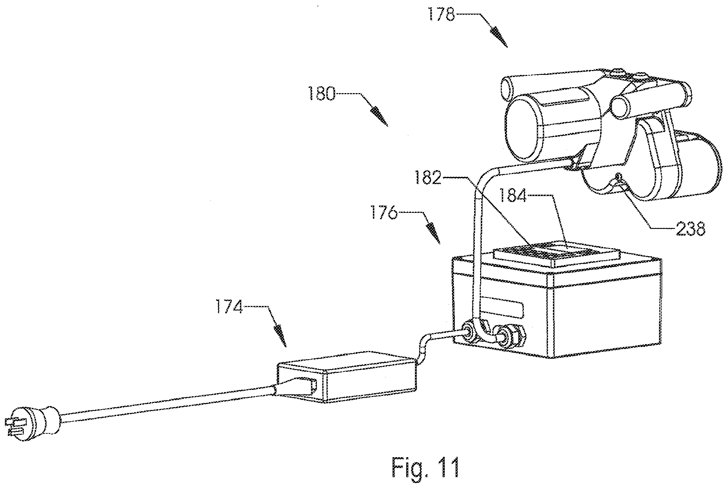

Turning to FIGS. 7 through 10, a bone reshaping implant having a magnetic drive is shown. The implant 110 has one or more distraction shaft screw holes 122 in the distraction shaft 114 through which the screws may be placed. Likewise, the housing 112 is attached to an end cap 130 which has one or more housing screw holes 124 through which the screws may be placed. The housing 112 of the bone reshaping implant 110 includes a magnet housing 128 and a splined housing 126. These housings 126, 128 may be attached to each other by means of welding, adhesive bonding or other joining techniques. The magnet housing 128 is sealably closed at one end (the end opposite the interface with the splined housing 126 by the attachment of the end cap 130. The end cap 130 may be attached to the magnet housing 128 by means of welding, adhesive bonding or other joining techniques. In use, the distraction shaft 114 is driven from the housing 112 by means of a lead screw 136 which turns inside a nut 140 that is secured to an inner surface adjacent to a cavity 137 of the distraction shaft 114. The lead screw 136 is mechanically coupled, in an indirect manner, to cylindrical permanent magnet 134 contained within the magnet housing 128. As explained in more detail below, rotation of the cylindrical permanent magnet 134, which is magnetically driven by an external adjustment device 180 (FIG. 11), effectuates rotation of the lead screw 136.

Cylindrical magnet 134 is fixedly contained within a magnet casing 158 using, for example, an adhesive such as an epoxy. The magnet casing 158 rotates relative to the magnet housing 128. The cylindrical magnet 134 may be a rare earth magnet such as Nd--Fe--B and may be coated with Parylene or other protective coatings in addition to being protected within the magnet casing 158, for example hermetically potted with epoxy. The magnet casing 158 contains an axle 160 on one end which attaches to the interior of a radial bearing 132. The outer diameter of the radial bearing 132 is secured to the interior of the end cap 130. This arrangement allows the cylindrical magnet 134 to rotate with minimal torsional resistance. At its other, opposing end, the magnet casing 158 includes an axle 161, which is attached to a first planetary gear set 154. The axle 161 includes the sun gear of the first planetary gear set 154, the sun gear turning the planetary gears of the first planetary gear set 154. The first planetary gear set 154 serves to reduce the rotational speed and increase the resultant torque delivery from the cylindrical magnet 134 to the lead screw 136. A second planetary gear set 156 is also shown between the first planetary gear set 154 and the lead screw 136, for further speed reduction and torque augmentation. The number of planetary gear sets and/or the number of teeth in the gears may be adjusted, in order to achieve the desired speed and torque delivery. For example, a lead screw with a pitch of 0.318 mm (eighty (80) threads per inch) attached to two planetary gear sets of 4:1 gear ratio each inside a 9 mm device with magnet location in the distal femur can achieve at least 445 N (100 lb). of distraction force at a greater than average distance or gap from the external device. The planetary gear sets 154, 156 output to a planetary gear output shaft 144. The planetary gear output shaft 144 extends through a thrust bearing 138 and is secured (by welding and the like) to a lead screw coupling cap 146. The lead screw 136 is secured to the lead screw coupling cap 146 by a locking pin 142, which extends through a hole in the lead screw 136 and holes in the lead screw coupling cap 146. A locking pin retainer 148 is a cylinder that surrounds the locking pin 142, holding this assembly together. Attaching the lead screw 136 to the rest of the magnet/gear assembly in this manner, assures that the design is not over-constrained, and thus that the lead screw 136 does not gall with the nut 140. In addition, a biocompatible grease, for example KRYTOX, may be used on the moving parts (lead screw, nut, bearings, housing, and distraction shaft) in order to minimize frictional losses. The lead screw 136 is able to freely rotate within a cavity 137 of the distraction shaft 114, and only need engage with the short length of the nut 140, this feature also minimizing frictional losses.

The thrust bearing 138 serves to protect the magnet/gear assembly of the drive from any significant compressive or tensile stresses. The thrust bearing 138 consists of two separate races with ball bearings between the two races. When there is a compressive force on the device, for example, when distracting a bone 100, and thus resisting the tensile strength of the soft tissues, the thrust bearing 138 abuts against a magnet housing abutment or lip 150 located in the magnet housing 128. Additionally, though the device is not typically intended for pulling bones together, there may be some applications where this is desired. For example, in certain compressive nail applications it is the goal to hold two fractured sections of a bone together. Because the bone 100 in an application involving an osteotomy or trauma may have fractured in a non-uniform or shattered pattern, it may be difficult to determine the desired length of the nail until after it is implanted and fully attached. In these situations, it can be easy to misjudge the length, and so a gap may exist between the bones. By placing a slightly extended intramedullary device 110 and securing it, the device 110 may be retracted magnetically, after it has been secured within the bone fragments, so that it applies the desired compression between the two fragments. In these compressive nail applications, there would be tensile force on the device 110 and the thrust bearing 138 would abut against a splined housing abutment or lip 152. In both situations, the thrust bearing 138 and a rigid portion of one of the housing sections take the large stresses, not the magnet/gear assembly of the drive system. In particular, the thrust bearing 138 is sandwiched between the abutment or lip 150 and the abutment or lip 152.

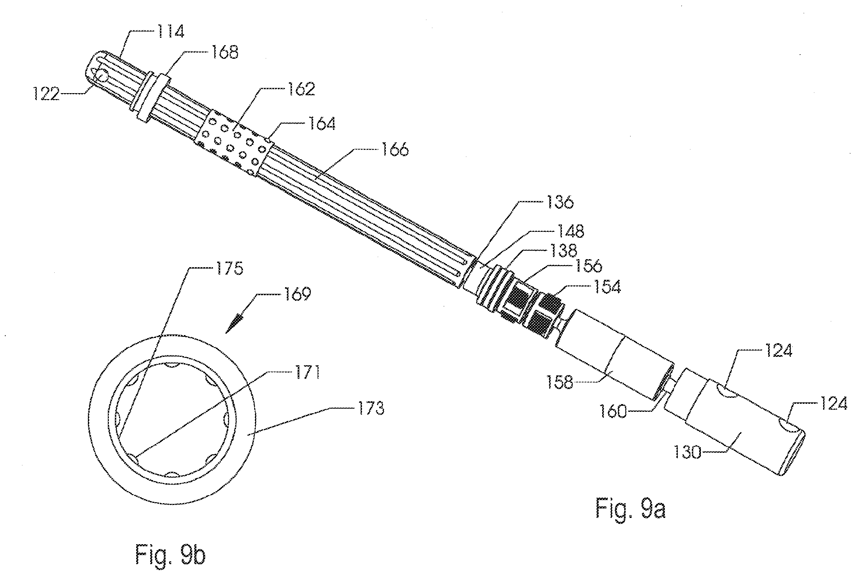

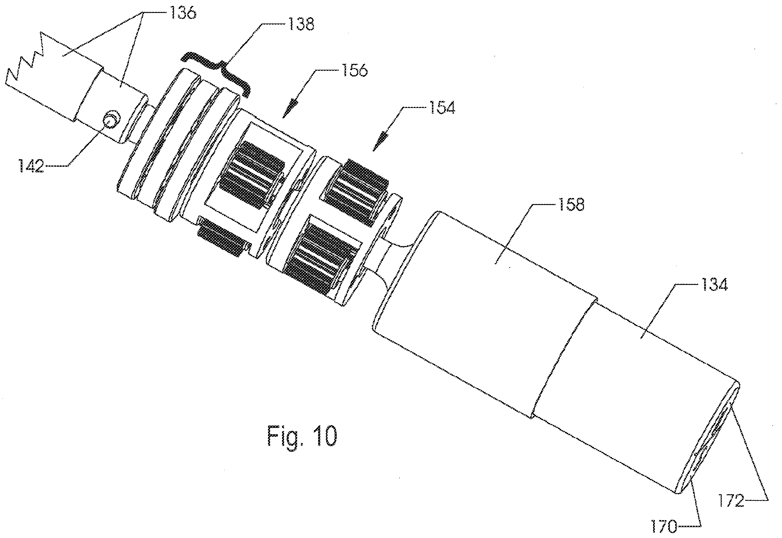

Turning specifically to FIGS. 8a and 9a, the housing components have been removed to reveal various internal features, including a collar that allows sliding of the distraction shaft 114 within the housing 112, and which also keeps the distraction shaft 114 from being able to rotate within the housing 112. This allows full stability of the bone 100. Distraction shaft 114 contains several axial grooves 166. The grooves 166 have semi-circular indentation cross-sections which allow several balls 164 to roll within them. The balls 164 are trapped within a linear ball cage 162. The splined housing 126 which fits over the balls 164 and linear ball cage 162 has axial grooves 163 (FIG. 8c) along its inner diameter surface that are similar to the axial grooves 166 of the distraction shaft 114. In this regard, the balls 164 and the ball cage 162 are interposed between the distraction shaft 114 and the splined housing 126. Therefore, the balls 164 are held in place by the linear ball cage 162, and mechanically lock the respective grooves to each other, thus impeding rotation of the distraction shaft 114 within the housing 112. However, the balls 164 are able to roll within the linear ball cage 162, thus allowing axial displacement of the distraction shaft 114 in relation to the splined housing 126 of the housing 112 with very low friction. A lip seal flange 168 contains a custom cross-section lip seal 169 (shown in FIG. 9b) which allows a sliding seal between the distraction shaft 114 and the splined housing 126, thus protecting the inner contents of the entire assembly from the body environment. The lip seal 169 includes a base portion 173, which seals against the inner diameter of the lip seal flange 168 (and thus the splined housing 126 which is attached to the lip seal flange 168). The lip seal 169 also includes protrusions 171 which slidingly seal against the axial grooves 166 of the distraction shaft 114. Inner surface 175 of the lip seal 169 slidingly seals against the overall outer diameter of the distraction shaft 114. It should also be noted that the lip seal 169 may be made from silicone, EPDM or other rubber materials, and may be coated with silicone oil, to aid in lubricity. Also, the balls, grooves and ball cage may be coated with silicone oil or a liquid perfluorinated polyether such as KRYTOX to aid in lubricity. FIG. 10 shows a portion of the magnet casing 158 removed so that the South pole 170 and North pole 172 of the cylindrical magnet 134 may be illustrated.

FIG. 11 illustrates an external adjustment device 180 which is used to non-invasively control the bone reshaping implant 110 by means of a magnetic coupling which transmits torque. The external adjustment device 180 comprises a magnetic handpiece 178, a control box 176 and a power supply 174. The control box 176 includes a control panel 182 having one or more controls (buttons, switches or tactile, motion, audio or light sensors) and a display 184. The display 184 may be visual, auditory, tactile, the like or some combination of the aforementioned features. The external adjustment device 180 may contain software which allows programming by the physician. For example, the physician may desire that the patient take home the external adjustment device 180 in order that the patient or member of the patient's family or friends make daily adjustments of the bone reshaping implant 110 implanted in the patient. However, the physician is able to keep the person operating the external adjustment device 180 from over adjusting the patient by programming this into the control box 176. For example, the physician may pre-program the control 176 box so that only one (1) mm of or other adjustment is allowed per day. The physician may additionally pre-program the control box 176 so that no more than 0.5 mm may be adjusted during any two hour period, or that no more than 0.25 mm may be adjusted during a five minute period. Settings such as these may serve to assure that the patient not be capable of causing severe damage to the bone or tissue, nor disrupt the bone reshaping process.

Preferably, such instructions or limits may be pre-programmed by the physician or even the manufacturer in a secure fashion such that user cannot alter the pre-programmed setting(s). For example, a security code may be used to pre-program and change the daily adjustment limit (or other parameters). In this example, the person operating the external adjustment device 180 will not be able to adjust more than one (1) mm in a day (or more than two mm in a day), and will not have the security code to be able to change this function of the external adjustment device 180. This serves as a useful lockout feature to prevent accidental over-adjustment of the bone reshaping implant 110. The safety feature may monitor, for example, rotational movement of magnets 186 of the external adjustment device 180 or the safety feature may monitor rotation of the cylindrical magnet 134 in the bone reshaping device 110, via non-invasive sensing means.

It is contemplated that various combinations or subcombinations of the specific features and aspects of the embodiments disclosed above may be made and still fall within one or more of the inventions. Further, the disclosure herein of any particular feature, aspect, method, property, characteristic, quality, attribute, element, or the like in connection with an embodiment can be used in all other embodiments set forth herein. Accordingly, it should be understood that various features and aspects of the disclosed embodiments can be combined with or substituted for one another in order to form varying modes of the disclosed inventions. Thus, it is intended that the scope of the present inventions herein disclosed should not be limited by the particular disclosed embodiments described above. Moreover, while the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents, and alternatives falling within the scope of the various embodiments described and the appended claims. Any methods disclosed herein need not be performed in the order recited. The methods disclosed herein include certain actions taken by a practitioner; however, they can also include any third-party instruction of those actions, either expressly or by implication. For example, actions such as "attaching a bone reshaping device to the bone" include "instructing the attaching a bone reshaping device to the bone." The ranges disclosed herein also encompass any and all overlap, sub-ranges, and combinations thereof. Language such as "up to," "at least," "greater than," "less than," "between," and the like includes the number recited. Numbers preceded by a term such as "approximately", "about", and "substantially" as used herein include the recited numbers, and also represent an amount close to the stated amount that still performs a desired function or achieves a desired result. For example, the terms "approximately", "about", and "substantially" may refer to an amount that is within less than 10% of, within less than 5% of, within less than 1% of, within less than 0.1% of, and within less than 0.01% of the stated amount.

## Claims

1. A bone reshaping implant (110) configured for reshaping a bone without creating an osteotomy, wherein the bone reshaping implant (110) is configured to impart a bone reshaping force on the bone (10), the bone reshaping implant (110) comprising:
a distraction shaft (114) configured to be attached to a first location on the bone and to apply the bone reshaping force onto the bone, the distraction shaft (114) having a nut (140) disposed on an inner surface adjacent to a cavity (137);
a housing (112) configured to be attached to a second location on the bone and to apply the bone reshaping force onto the bone, the housing (112) further comprising:
a cylindrical permanent magnet (134) configured to be noninvasively actuated to generate the bone reshaping force, the cylindrical permanent magnet (134) fixedly contained within a magnet casing (158), the magnet casing (158) further comprising an axle (161) which is configured to engage at least one planetary gear set (154),
a lead screw mechanically coupled to the cylindrical permanent magnet (134) and configured to communicate with the nut (140) of the distraction shaft (114) to drive the distraction shaft (114) relative to the housing (112);
wherein the cylindrical permanent magnet (134) and at least one planetary gear set (154) are rotatably disposed on a thrust bearing (138) and wherein the thrust bearing (138) is configured to protect the cylindrical permanent magnet (134) and at least one planetary gear set (154) from at least one of compressive or tensile stresses.

2. The bone reshaping implant (110) of claim 1 wherein the bone reshaping force comprises at least an axially compressive force component on the bone.

3. The bone reshaping implant (110) of claim 1 wherein the bone reshaping force comprises at least an axial tension force component on the bone.

4. The bone reshaping implant (110) of claim 1 wherein the bone reshaping force comprises at least a torsional force component on the bone.

5. The bone reshaping implant (110) of claim 1 wherein the bone reshaping force comprises at least a lateral bending force component on the bone.

6. The bone reshaping implant (110) of claim 4 wherein the torsional force is in the range of about (25-75 in·lbs) 2.825-8.475 Nm.

7. The bone reshaping implant (110) of claim 1 wherein the cylindrical permanent magnet (134) is configured to be activated by a wireless controller outside of the patient.

8. The bone reshaping implant (110) of claim 1 wherein at least one of the housing (112) and the distraction shaft (114) comprises an aperture configured to receive a bone screw (101, 102).

9. The bone reshaping implant (110) of claim 1 wherein the bone reshaping force comprises at least: an axially compressive force component on the bone, or a torsional force component on the bone, or a lateral bending force component on the bone.

## Patentansprüche

1. Knochenumformungsimplantat (110), das konfiguriert ist, um ohne Vornahme einer Osteotomie einen Knochen umzuformen, wobei das Knochenumformungsimplantat (110) konfiguriert ist, um eine Knochenumformkraft auf den Knochen (10) auszuüben, wobei das Knochenumformungsimplantat (110) umfasst:
eine Distraktionswelle (114), die konfiguriert ist, um an einer ersten Stelle am Knochen befestigt zu werden und die Knochenumformkraft auf den Knochen auszuüben, wobei die Distraktionswelle (114) eine Mutter (140) aufweist, die auf einer Innenfläche benachbart zu einem Hohlraum (137) angeordnet ist;
ein Gehäuse (112), das konfiguriert ist, um an einer zweiten Stelle am Knochen befestigt zu werden und die Knochenumformkraft auf den Knochen auszuüben, wobei das Gehäuse (112) ferner umfasst:
einen zylindrischen Permanentmagneten (134), der konfiguriert ist, um nicht-invasiv betätigt zu werden, um die Knochenumformkraft zu erzeugen, wobei der zylindrische Permanentmagnet (134) fest in einem Magnetgehäuse (158) enthalten ist, wobei das Magnetgehäuse (158) ferner eine Achse (161) umfasst, die konfiguriert ist, um mit mindestens einem Planetenradsatz (154) in Eingriff zu sein,
eine Leitspindel, die mechanisch mit dem zylindrischen Permanentmagneten (134) gekoppelt und konfiguriert ist, um mit der Mutter (140) der Distraktionswelle (114) zu kommunizieren, um die Distraktionswelle (114) relativ zum Gehäuse (112) anzutreiben;
wobei der zylindrische Permanentmagnet (134) und der mindestens eine Planetenradsatz (154) drehbar auf einem Axiallager (138) angeordnet sind und wobei das Axiallager (138) konfiguriert ist, um den zylindrischen Permanentmagneten (134) und den mindestens einen Planetenradsatz (154) vor mindestens einer von Druck- oder Zugspannungen zu schützen.

2. Knochenumformungsimplantat (110) nach Anspruch 1, bei dem die Knochenumformungskraft mindestens eine axial zusammendrückende Kraftkomponente auf den Knochen umfasst.

3. Knochenumformungsimplantat (110) nach Anspruch 1, bei dem die Knochenumformungskraft mindestens eine axiale Zugkraftkomponente auf den Knochen umfasst.

4. Knochenumformungsimplantat (110) nach Anspruch 1, bei dem die Knochenumformungskraft mindestens eine Torsionskraftkomponente auf den Knochen umfasst.

5. Knochenumformungsimplantat (110) nach Anspruch 1, bei dem die Knochenumformungskraft mindestens eine laterale Biegekraftkomponente auf den Knochen umfasst.

6. Knochenumformungsimplantat (110) nach Anspruch 4, bei dem die Torsionskraft im Bereich von etwa (25-75 in-lbs) 2,825-8,475 Nm liegt.

7. Knochenumformungsimplantat (110) nach Anspruch 1, bei dem der zylindrische Permanentmagnet (134) konfiguriert ist, um durch eine drahtlose Steuerung außerhalb des Patienten aktiviert zu werden.

8. Knochenumformungsimplantat (110) nach Anspruch 1, bei dem von dem Gehäuse (112) und der Distraktionswelle (114) mindestens eines eine Öffnung aufweist, die zur Aufnahme einer Knochenschraube (101, 102) ausgebildet ist.

9. Knochenumformungsimplantat (110) nach Anspruch 1, bei dem die Knochenumformungskraft mindestens umfasst: eine axial zusammendrückende Kraftkomponente auf den Knochen oder eine Torsionskraftkomponente auf den Knochen oder eine laterale Biegekraftkomponente auf den Knochen.

## Revendications

1. Implant de refaçonnage osseux (110) configuré pour refaçonner un os sans créer d'ostéotomie, dans lequel l'implant de refaçonnage osseux (110) est configuré pour imprimer une force de refaçonnage osseux sur l'os (10), l'implant de refaçonnage osseux (110) comprenant :
une tige de distraction (114) configurée pour être attachée à un premier emplacement sur l'os et pour appliquer la force de refaçonnage de l'os sur l'os, la tige de distraction (114) ayant un écrou (140) disposé sur une surface interne adjacente à une cavité (137) ;
un boîtier (112) configuré pour être attaché à un second emplacement sur l'os et pour appliquer la force de refaçonnage osseux sur l'os, le boîtier (112) comprenant en outre :
un aimant permanent cylindrique (134) configuré pour être actionné de manière non invasive pour générer la force de refaçonnage osseux, l'aimant permanent cylindrique (134) contenu de manière fixe à l'intérieur d'une enveloppe magnétique (158), l'enveloppe magnétique (158) comprenant en outre un axe (161) qui est configuré pour venir en prise avec au moins un ensemble d'engrenage planétaire (154),
une vis-mère couplée mécaniquement à l'aimant permanent cylindrique (134) et configurée pour communiquer avec l'écrou (140) de la tige de distraction (114) afin d'entraîner la tige de distraction (114) par rapport au boîtier (112) ;
dans lequel l'aimant permanent cylindrique (134) et au moins un ensemble d'engrenage planétaire (154) sont disposés de manière rotative sur un palier de poussée (138) et dans lequel le palier de poussée (138) est configuré pour protéger l'aimant permanent cylindrique (134) et au moins un ensemble d'engrenage planétaire (154) provenant d'au moins une de contraintes de compression ou de traction.

2. Implant de refaçonnage osseux (110) selon la revendication 1, dans lequel la force de refaçonnage osseux comprend au moins une composante de force de compression axiale sur l'os.

3. Implant de refaçonnage osseux (110) selon la revendication 1, dans lequel la force de refaçonnage osseux comprend au moins une composante de force de traction axiale sur l'os.

4. Implant de refaçonnage osseux (110) selon la revendication 1, dans lequel la force de refaçonnage osseux comprend au moins une composante de force de torsion sur l'os.

5. Implant de refaçonnage osseux (110) selon la revendication 1, dans lequel la force de refaçonnage osseux comprend au moins une composante de force de flexion latérale sur l'os.

6. Implant de refaçonnage osseux (110) selon la revendication 4, dans lequel la force de torsion est dans la plage d'environ 2,825 à 8,475 Nm (25 à 75 in-lbs).

7. Implant de refaçonnage osseux (110) selon la revendication 1, dans lequel l'aimant permanent cylindrique (134) est configuré pour être activé par une commande sans fil à l'extérieur du patient.

8. Implant de refaçonnage osseux (110) selon la revendication 1, dans lequel au moins l'un du boîtier (112) et de la tige de distraction (114) comprend une ouverture configurée pour recevoir une vis à os (101, 102).

9. Implant de refaçonnage osseux (110) selon la revendication 1, dans lequel la force de refaçonnage osseux comprend au moins : une composante de force de compression axiale sur l'os, ou une composante de force de torsion sur l'os, ou une composante de force de flexion latérale sur l'os.
